# EUROPEAN PATENT APPLICATION

(11) **EP 3 659 554 A1**
(43) Date of publication of application: **03.06.2020**
(21) Application number: 18209130.6
(22) Date of filing: 29.11.2018
(51) Int. Cl.: A61F 2/30, A61F 2/38, A61F 2/00, A61B 17/68, A61B 17/064

(54) **AN ANCHORING PIN**

(71) Applicant: The Provost, Fellows, Scholars and other Members of Board of Trinity College Dublin, Dublin 2 (IE)
(72) Inventor: Kelly, Daniel John, Dublin 2, (IE); Browe, David Colin, Dublin 2, (IE); Burdis, Ross, Dublin 2, (IE)
(74) Representative: Purdylucey Intellectual Property

(57) **Abstract**

A biocompatible and biodegradable anchoring pin 1 made up of an elongate shaft 2 for insertion in implants such as biomaterial based scaffolds 13 and the like or engineered tissues, and an integral circular head 3 on the shaft 2 for holding the implants in position at musculoskeletal defects in tissues such as bone, tendon, cartilage and ligament.

## Description

### Introduction

This invention relates to an anchoring pin and more particularly to a biocompatible anchoring pin for implant fixation. The invention also relates to an implant retention system comprising a combined anchoring pin and implant and to a method of regenerating cartilage in a patient employing the anchoring pin and an implant scaffold.

### Background of the Invention

Musculoskeletal injuries and chondral defects can be difficult to treat due to the anatomical shape of and biomechanical forces present in synovial joints such as the knee. For example, where defects result from tissue degeneration such as cartilage failure, implants are employed for tissue regeneration. Examples of such implants are tissue engineered constructs or biodegradable scaffolds formed from extracellular matrix (ECM) which are implanted into chondral defects and integrate with host tissue to regenerate cartilage. Examples of scaffolds are to be found in European Patent Specification No. 3180043A1.

Primary fixation of scaffolds or tissue engineered constructs is of vital importance as without stable fixation the implanted construct will not successfully integrate with the host tissue resulting in graft failure and poor clinical outcome. Due to the anatomical shape and biomechanical forces within synovial joints such as the knee, the fixation of scaffolds into joint defects is a major issue for the field. Accordingly, it is often necessary to suture scaffolds into cartilage defects which is a very time consuming and technically challenging surgical procedure reliant on surgical skill.

In order to promote tissue regeneration, scaffolds can be loaded with tissue cells. However, known scaffolds must be pre-seeded with tissue cells prior to implantation which can be a time consuming and expensive additional step in the treatment of defects.

As an alternative to the use of scaffold implants, it is also known to employ microfracture or microdrilling in which minute fractures are formed in bone which give rise to cartilage regeneration. In microfracture a pick-like surgical tool is employed to form a network of holes in bone which ultimately result in the formation of fibrocartilage at the site. In microdrilling, a drill bit is used to form the holes. Microfracture/microdrilling is often the first line of clinical defence employed to treat focal cartilage defects. However, the medium-long term efficacy of this surgical approach is limited.

It is also known to use fixation devices in musculoskeletal procedures. However, in general, such devices are poorly compatible with implant devices such as tissue regeneration scaffolds and/or are made up of separate components which can be expensive and difficult to produce in the dimensions required for use with implant devices such as scaffolds. For example, US Patent Specification No. 2008/0051796 A1 describes a graft retention tack for use in the repair of articular cartilage. However, the graft retention tack is made up of multiple separate elements such as a post and cap(s) which must be manufactured separately and assembled in use.

### Summary of the Invention

According to the invention there is provided a biocompatible implant anchoring pin comprising:
a shaft for insertion in tissue and
an integral head on the shaft configured to retain an implant in tissue.

Preferably, the head comprises an implant retainer and in a preferred embodiment the implant retainer comprises a scaffold implant retainer. Preferably, the implant retainer comprises an ECM scaffold implant retainer. Alternatively, the implant retainer comprises an engineered tissue implant retainer.

Advantageously, the implant retainer is apertured. Preferably, the apertured implant retainer comprises at least one relatively large aperture to allow passage of biological matter through the implant retainer. Alternatively, the apertured implant retainer comprises relatively small apertures within which blood can clot or tissue can form. Suitably, the relatively small apertures are defined by a regular array and, advantageously, the relatively small apertures comprise microchambers in the regular array.

Preferably, the shaft comprises a proximal end at the integral head, a distal end remote from the integral head and a lumen defined in the shaft extending between the proximal end and the distal end. Suitably, the distal end comprises a distal end tip for insertion into tissue.

Preferably, the shaft comprises tissue grips. More preferably, the tissue grips comprise laterally extending ridges.

Suitably, the anchoring pin comprises a spacer to space the integral head from tissue. Preferably, the spacer comprises a chamfer on the shaft.

In one embodiment, the shaft comprises tissue specific or tailored zones having different physical, chemical or biological properties in accordance with tissue type. Preferably, the tissue specific zones have different stiffnesses adapted for penetrating different tissues.

In an alternative embodiment of the invention, the anchoring pin comprises a plurality of shafts.

In a preferred embodiment of the invention, the anchor pin is porous.

Preferably, the anchoring pin comprises a biodegradable polymer. More preferably, the biodegradable polymer comprises polycaprolactone and/or poly(lactic-co-glycolic acid).

Suitably, if desired, the polymers can also be augmented, doped or coated with other materials to alter the physical, chemical or biochemical properties of the anchoring pin. Preferably, the polymer is augmented, doped or coated with an osteoinductive material. More preferably, the osteoinductive material comprises hydroxyapatite. Suitably, the anchoring pin comprises solubilised bone and/or articular cartilage ECM. Optionally, the anchoring pin comprises a chemoattractant and/or a drug. Preferably, the chemoattractant comprises (TGF)-β3.

Advantageously, the anchoring pin is formed by a continuous additive manufacturing technique. Preferably, the continuous additive manufacturing technique comprises a 3D printing technique.

The invention also extends to an implant retention system comprising an anchoring pin as hereinbefore defined combined with an implant. Preferably, the implant comprises a scaffold. More preferably, the scaffold comprises an ECM scaffold.

Suitably, the anchoring pin is combined with the scaffold by insertion of the anchoring pin in the scaffold. Alternatively, the anchoring pin is combined with the scaffold in an integrated unitary structure.

In further embodiment, the invention extends to a method for manufacturing a conjoined anchoring pin and scaffold implant comprising first forming an anchoring pin as hereinbefore defined and then forming a scaffold on the anchoring pin. Preferably, the scaffold implant is formed on the anchoring pin by lyophilisation.

The invention also extends to a method of regenerating cartilage in a patient comprising:
creating a microfracture/microdrilled hole in bone and
securing a scaffold at the hole with a biocompatible anchoring pin having a shaft for insertion in the hole and an integral head on the shaft configured to retain the scaffold in the hole.

Preferably, the integral head of the anchoring pin comprises a scaffold retainer.

Suitably, the scaffold retainer is apertured and, preferably, the apertured scaffold retainer comprises at least one relatively large aperture to allow passage of biological matter through the implant retainer.

Alternatively, the apertured implant retainer comprises relatively small apertures within which blood can clot or tissue can form. Preferably, the relatively small apertures are defined by a regular array. Advantageously, the relatively small apertures comprise microchambers in the regular array.

Preferably, the shaft comprises a proximal end at the integral head, a distal end remote from the integral head and a lumen defined in the shaft extending between the proximal end and the distal end.

Suitably, the distal end comprises a distal end tip for insertion into bone.

Preferably, the shaft comprises tissue grips. More preferably, the tissue grips comprise laterally extending ridges.

Preferably, the method comprises the step of spacing the head of the anchoring pin from the bone so that the head is recessed or parallel with respect to surrounding cartilage. More preferably, the head of the anchoring pin is spaced from the bone with a spacer on the shaft. Most preferably, the spacer comprises a chamfer on the shaft.

In one embodiment, the microfracture/microdrilled hole is located in bone with a template.

In one embodiment of the method of the invention, the shaft comprises tissue specific or tailored zones having different physical, chemical or biological properties in accordance with tissue type. Preferably, the tissue specific zones have different stiffnesses adapted for penetrating different tissues.

In another embodiment of the invention, the anchoring pin comprises a plurality of shafts.

Preferably, the anchoring pin is porous. More preferably, the anchoring pin comprises a biodegradable polymer. Most preferably, the biodegradable polymer comprises polycaprolactone and/or poly(lactic-co-glycolic acid).

Suitably, if desired, the method comprises augmenting, doping or coating the polymer with other materials to alter the physical, chemical or biochemical properties of the anchoring pin. Preferably, the polymer is augmented, doped or coated with an osteoinductive material. More preferably, the osteoinductive material comprises hydroxyapatite.

Optionally, the anchoring pin comprises solubilised bone and/or articular cartilage ECM. Suitably, the anchoring pin comprises a chemoattractant and/or a drug and, preferably, the chemoattractant comprises (TGF)-β3.

In a preferred embodiment of the method of regenerating cartilage in a patient, the scaffold comprises an ECM scaffold.

The biodegradable anchoring pin of the invention can be fabricated using continuous additive manufacturing techniques such as 3D printing. The anchoring pin is a fixation device for implants such as biomaterial based scaffolds or engineered tissues used to treat musculoskeletal injuries. In addition to defects located in knee joints, the anchoring pins of the invention can also be used to fixate scaffolds as part of anchoring pin - scaffold implant combinations in a range of diverse musculoskeletal tissues such as bone, tendon and ligament.

The anchoring pin enables effective and optimal scaffold fixation by providing a fixation device that maintains a scaffold and/or engineered tissue in position while degrading overtime once repair tissue has formed. The anchoring pin can be adapted for use with known scaffolds of varying shapes, sizes and materials.

Where the anchoring pin of the invention is conjoined with a scaffold or similar implant to form a unified anchoring pin - scaffold implant combination joint regeneration is optimally promoted.

The anchoring pin and anchoring pin - scaffold implant combination of the invention therefore assists in tissue (e.g. articular cartilage) regeneration that is targeted at recruiting endogenous bone marrow derived stem cells into a porous scaffold.

The anchoring pin can serve to anchor or securely fixate porous scaffolds to bone and is particularly suited to the regeneration of articular cartilage. However, the anchoring pin of the invention is more generally suitable for use in other soft-to-hard tissue fixation applications (e.g. tendon-bone) or for the fixation of scaffolds designed to regenerate large bone defects.

Due to the unitary structure of the anchoring pin, the anchoring pin of the invention polymer pin can be fabricated using 3D printing which has the benefits of being a continuous, additive manufacturing technique.

Accordingly, as a modular structure, the anchoring pin can be employed as a standalone pin for use in soft-to-hard tissue fixation scenarios, in combination with commercially treatment methods or in conjunction with scaffolds and engineered tissues. Where the anchoring pin is employed with scaffolds, the scaffold and anchoring pin can be connected or assembled in use after both have been fully fabricated (e.g. by pushing the pin through softer ECM scaffolds), or by freeze-drying the ECM scaffold directly to the head of the pin to form a fully integrated anchoring pin - scaffold combination. Both approaches are effective so that the anchoring pin can function as a standalone or combinational off the shelf medical device.

In summary, the anchoring pin and anchoring pin - scaffold combination of the invention therefore offer a number of significant benefits over existing cell and biomaterial based approaches for articular cartilage regeneration. Firstly, the need for pre-seeding scaffolds with cells prior to implantation is obviated, and, secondly, the need for suturing of a scaffold into a cartilage defect, which is a very time consuming and technically challenging surgical procedure, is also obviated. The anchoring pin of the invention serves to ensure effective primary stable fixation of scaffolds or tissue engineered constructs for successful integration with host tissue resulting in optimal grafting and positive clinical outcomes. Thirdly, the anchoring pin and anchoring pin - scaffold combination can be used in combination with microfracture/microdrilling cartilage regeneration treatment methods to provide a new microfracture/microdrilling cartilage regeneration treatment method that enhances successful outcomes with microfracture/microdrilling cartilage regeneration treatments which are often the first line of defence clinically. Finally, improved performance is achieved with the anchoring pins of the invention in combination with the inherent chondro-inductivity of the cartilage ECM derived scaffolds developed by the applicant and described in European Patent Specification No. 3180043A1 maximises the potential for hyaline cartilage regeneration.

### Brief Description of the Drawings

The invention will now be described, by way of example only, with reference to the accompanying drawings in which:
Figure 1 is a perspective view from above and the head end of two biocompatible and biodegradable anchoring pins of the invention made up of a circular head and a ridged shaft integrally attached to the circular head in a unitary anchoring pin structure;
Figure 2 is a side elevation of the anchoring pins of Figure 1 with the ridges on the shaft more clearly visible;
Figure 3 is a perspective view from above and one side of the anchoring pins with the shaft oriented in a vertically upwards position from the head;
Figure 4 is a side elevation of an anchoring pin gripped by a tool at its shaft;
Figure 5 is a macroscopic image of an anchoring pin implanted in a hole formed e.g. by drilling at a caprine chondral defect to secure an ECM scaffold at the defect;
Figure 6 is an enlarged schematic cross-sectional view of the anchoring pin of Figure 5 inserted in the hole at the defect with a chamfer formed on the shaft adjacent the head of the anchoring pin to space the head from bone tissue and prevent over insertion of the anchoring pin in the hole;
Figure 7 is a further enlarged schematic cross-sectional view of the anchoring pin head, chamfer, bone and cartilage of Figure 6 with the anchoring pin head recessed with respect to the cartilage surface to prevent rubbing of the anchoring pin and scaffold against adjacent tissue;
Figure 8 is a macroscopic image of the anchoring pin and scaffold of Figure 5 four months post implant illustrating significant cartilage growth;
Figure 9 is a macroscopic image of a second embodiment of the anchoring pin of the invention in which the aperture head has a mesh or grid-like structure within which clots can form or which can function as a scaffold or platform for forming articular-like cartilage *in vitro* prior to implantation;
Figure 10 is a macroscopic image of the anchoring pin of Figure 9 implanted into a caprine chondral defect in the trochlear ridge of a goat;
Figure 11 is a macroscopic image of the anchoring pin of Figure 9 in which the mesh or grid-like head contains tissue engineered cartilage micro-pellets;
Figure 12 is a macroscopic image of the anchoring pin of Figure 11 implanted into a caprine chondral defect in the trochlear ridge of a goat;
Figure 13 is a macroscopic image and associated schematic drawing of the anchoring pin of Figures 1 to 8 and an ECM scaffold;
Figure 14 is a macroscopic image from above and associated schematic drawing of the anchoring pin and ECM scaffold of Figure 13 combined with the anchoring pin inserted in the ECM scaffold to secure the ECM scaffold *in situ* as required;
Figure 15 is a macroscopic image form the side and associated schematic drawing of the anchoring pin and scaffold of Figure 14;
Figure 16 is an enlarged schematic representation of the ECM scaffold illustrating tissue regeneration within the scaffold;
Figure 17 is a schematic representation of a first step in the manufacture of an integrated or unified anchoring pin-ECM scaffold combination in which an anchoring pin of Figures 1 to 8 is placed in a scaffold mould with the shaft of the anchoring pin oriented vertically upwards;
Figure 18 is a schematic representation of the mould and anchoring pin of Figure 17 with solubilised ECM slurry charged in the mould prior to lyophilisation;
Figure 19 is a schematic representation of the mould of Figure 18 post-lyophilisation with the ECM scaffold lyophilised around and to the anchoring pin to form the conjoined anchoring pin-ECM scaffold construct;
Figure 20 is a side elevation of another embodiment of the anchoring pin of the invention in which the anchoring pin is provided with three porous shafts and the head is mesh- or regular array-like in structure;
Figure 21 is a side elevation of the anchoring pin of Figure 20 in combination with a conjoined integrated ECM scaffold similar to the anchoring pin-ECM scaffold construct of Figure 19, and
Figure 22 is a schematic representation of the anchoring pin-ECM scaffold construct of Figure 21 implanted *in situ* in a defect at articular cartilage (AC) and calcified cartilage (CC).

### Detailed Description of the Invention

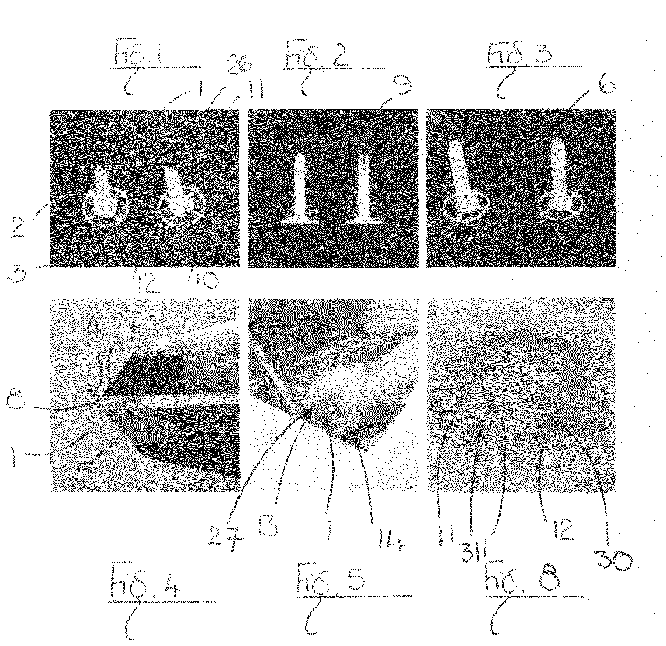

As shown in Figures 1 to 8, a first embodiment of a biocompatible and biodegradable anchoring pin of the invention is generally indicated by the reference numeral 1 and is made up of an elongate shaft 2 for insertion in implants such as biomaterial based scaffolds and tissues, and an integral circular head 3 on the shaft 2 for holding the implants in position in holes 27 formed at musculoskeletal defects in tissues such as bone, tendon and ligament. As discussed in more detail below, the anchoring pin can be formed from suitable biodegradable materials such as polymers e.g. polycaprolactone and can be sized and shaped as required.

The shaft 2 is integrally attached to the circular head 3 in a unitary anchoring pin structure and has a shaft proximal end 4 adjacent the integral head 3 and a shaft distal end 5 remote from the head 3 provided with a distal end tip 6 for insertion through implants and into tissue. The shaft 2 is provided with laterally extending tissue grips in the form of laterally extending ridges 7 along its length which allow for tissue integration and ensure a robust fixation within bone tissue and the like. In the present embodiment, the shaft 2 has a length of approximately 6mm and a diameter of about 1.58mm with a head diameter of about 6mm.

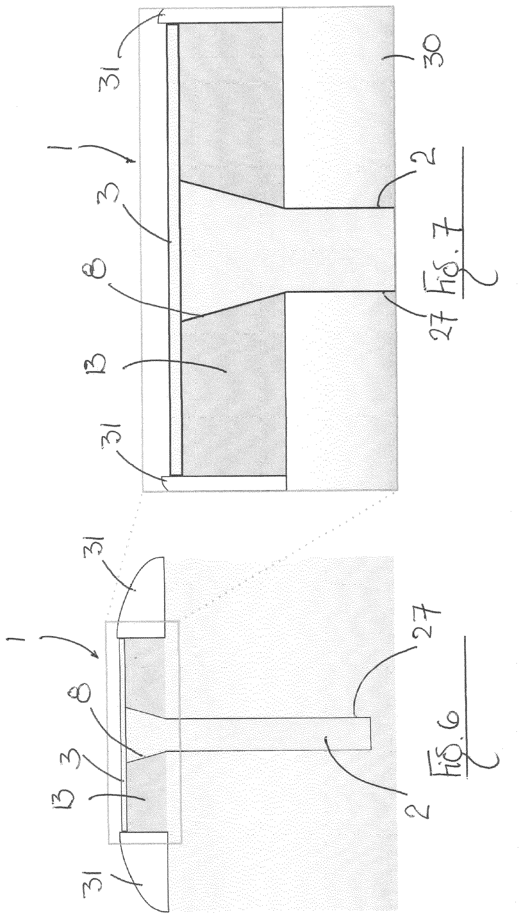

As shown particularly in Figures 6 and 7, the shaft 2 meets the head 3 at a chamfer 8 defined on the shaft 2 at its proximal end 4 adjacent the underside of the head 3. The chamfer 8 is shaped and dimensioned to define a stop 8 on the anchoring pin 1 to prevent the anchoring pin 1 from being over inserted in the hole 27 formed in tissue such as bone 30. This shall be explained more fully below. The chamfer 8 also reinforces the head 3 on the shaft 2 to in turn strengthen the unitary anchoring pin 1.

The shaft 2 is adapted to define a patency in the form of a bore or lumen 9 through the shaft 2 which can provide a conduit for fluids such as blood from subchondral bone and the like which can in turn deliver cells such as endogenous progenitor cells to defect or injury sites for regeneration.

The head 3 is an apertured head 3 defining an implant retainer 10 in the form of a substantially circular platform 10 on the chamfer 8 at the shaft 2 having a diameter greater than that of the chamfer 8 so that the circular platform 10 extends laterally outwards from the chamfer 8. The platform 10 is in turn surrounded by an outer ring 11 connected to the platform 10 by four circumferentially spaced apart support struts 12 extending between the platform 10 and the outer ring 11. The outer ring 11 and the support struts 12 serve to increase the surface area of the implant retainer 10 to increase the contact surface area of the implant retainer 10 with implants for optimal fixation while, importantly, the relatively large apertures 26 defined between the struts 12 allow the passage of biological matter such as nutrients through the implant retainer 10 of the apertured head 3 to aid tissue generation.

As shown particularly in Figures 5 to 8, the anchoring pin 1 is particularly adapted to secure implants in the form of ECM scaffolds 13 and the like in place in soft-to-hard tissue fixation scenarios. More particularly, Figure 5 shows an ECM scaffold 13 implanted in a caprine chondral defect 14 and secured in the chondral defect 14 with the anchoring pin 1 to fix the ECM scaffold 13 in the chondral defect 14. In the present embodiment, the implant retainer 10 therefore functions as a scaffold retainer 10 to hold the scaffold 13 in place in use.

As indicated above, the stop 8 formed by the chamfer 8 is sized to have a greater thickness or diameter than the shaft 2 so that the shaft 2 and hence the anchoring pin 1 is prevented from being over-inserted into the hole 27 i.e. the stop 8 can be proportioned as required relative to the size of the hole 27 to be formed in bone so that the anchoring pin 1 is prevented from being fully inserted in the bone. As shown in Figures 6 and 7, the stop 8 serves as a spacer to space the head 3 a desired distance from the surface of the bone 30 in which the shaft 2 is inserted. As a result, the head 3 can be recessed or positioned parallel with the (undamaged) cartilage 31 already present adjacent the chondral defect 14. Therefore, the anchoring pin 1, and in particular the head 3, can be prevented from abutting or rubbing against adjacent oppositely disposed tissues frequently found at joints. As will be appreciated by those skilled in the art, the relative and/or cumulative dimensions of the shaft 2, head 3 and chamfer 8 can be selected in accordance with the end use of the anchoring pin 1 and the required dimensions of the holes 27. For example, human cartilage can generally have a thickness of from about 2 to about 2.5mm so that a cumulative thickness of the head 3 and the chamfer 8 of about 2mm is considered suitable.

The anchoring pin 1 therefore serves to effectively hold the ECM scaffold 13 in place at the hole 27 defined in bone 30 during tissue regeneration over extended periods to optimise clinical outcomes as shown in Figure 8 which clearly shows successful cartilage 31 regeneration four months post implant of the ECM scaffold 13 with the anchoring pin 1.

As will be appreciated by those skilled in the art, in the implant retention system of the invention, a single relatively large implant such as an ECM scaffold 13 can be used with multiple anchoring pins 1 to hold the relatively large ECM scaffold 13 in place at a relatively large defect 14. Alternatively, multiple relatively smaller ECM scaffolds 13 can be employed at a relatively large defect 14 which are held in place by multiple anchoring pins 1 as required.

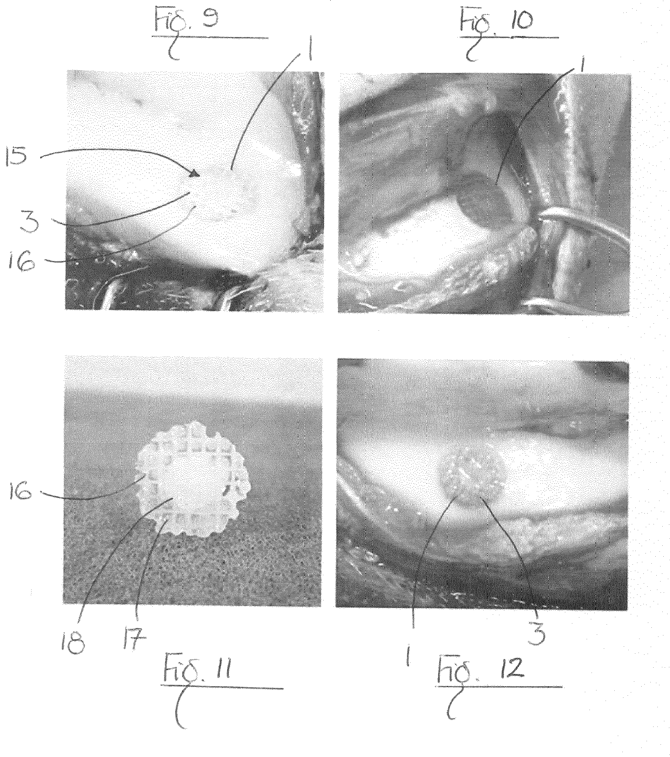

Figures 9 to 12 show a second embodiment of the anchoring pin 1 of the invention in which the implant retainer 10 of the head 3 is apertured in the form of a mesh or regular array structure 15 within which clots can form or which can function as a scaffold or platform for forming engineered tissue such as articular-like cartilage *in vitro* prior to implantation. More particularly, the platform-like implant retainer 10 is made up of perpendicularly disposed intersecting ribs 16 shaped and sized to define a grid of relatively small apertures in the form of individual cells 17 through which biological matter such as nutrients can pass or in which clots or engineered tissue can form or be formed as required. As shown in Figure 9, the individual cells 17 also facilitate the formation of corresponding individual engineered tissue pellets in the cells 17 *in vitro* prior to implantation to further optimise tissue regeneration.

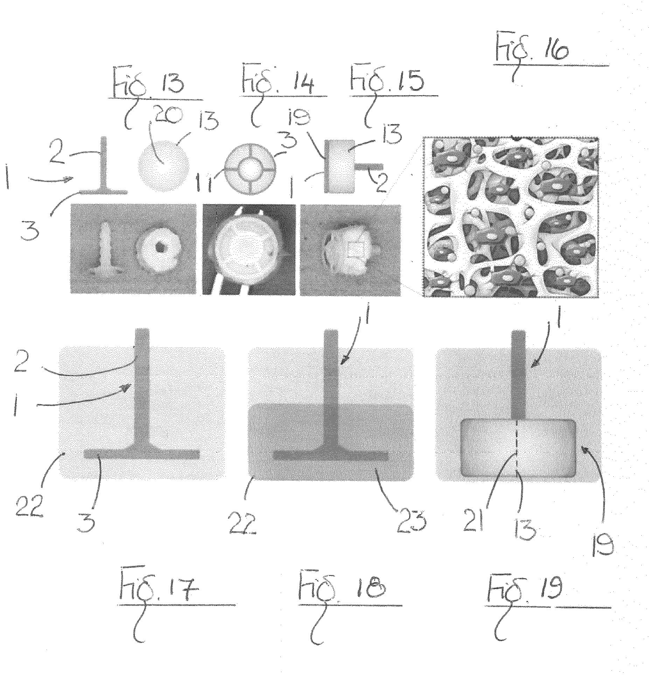

Figures 13 to 16 show the anchoring pin 1 of Figures 1 to 8 combined with an ECM scaffold 13 to form an anchoring pin - scaffold implant combination 19. As shown in the drawings, in the present embodiment, the ECM scaffold 13 is generally cylindrical in shape and the anchoring pin 1 is inserted in or received in a central bore 20 defined in the scaffold 13. The central bore 20 extends along the longitudinal axis 21 of the cylindrical scaffold 13 and the shaft 2 of the anchoring pin 1 is sized, shaped and dimensioned to pass through the central bore 20 while the implant retainer 10 of the head 3 of the anchoring pin 1 serves to abut the scaffold 13 to secure the scaffold 13 in place. The scaffold 13 is secured in a defect by inserting the distal end tip 6 into bone tissue and the like.

As shown in Figure 16, the anchoring pin - scaffold implant combination 19 facilitates optimal tissue regeneration within the scaffold 13 *in situ.*

As will be appreciated by those skilled in the art, the scaffold 13 can have varying shapes and sizes as required and the anchoring pin 1 can be sized, shaped and dimensioned in accordance with the scaffold 13 to form a highly effective anchoring pin - scaffold implant combination 19 for optimal tissue regeneration in which the anchoring pin 1 and scaffold 13 perform synergistically.

Figures 17 to 20 show a method of manufacturing of a fully integrated or unified anchoring pin-ECM scaffold combination 19 in which the anchoring pin 1 of Figures 1 to 8 is conjoinedly integrated with the scaffold 13. As shown in the drawings, a pre-formed anchor in 1 is first placed in a scaffold mould 22 with the shaft 2 of the anchoring pin 1 oriented vertically upwards. The scaffold mould 22 is shaped, sized and dimensioned in accordance with the desired configuration of the scaffold 13 while the pre-formed anchoring pin 1 is sized and shaped as required.

As shown in Figure 18, solubilised ECM slurry 23 is then charged into the mould 22 and then lyophilised to form the scaffold 13 element of the anchoring pin-ECM scaffold combination 19. More particularly, as shown in Figure 19, the ECM scaffold 13 is lyophilised around and to the anchoring pin 1 to form the conjoined anchoring pin-ECM scaffold construct 19.

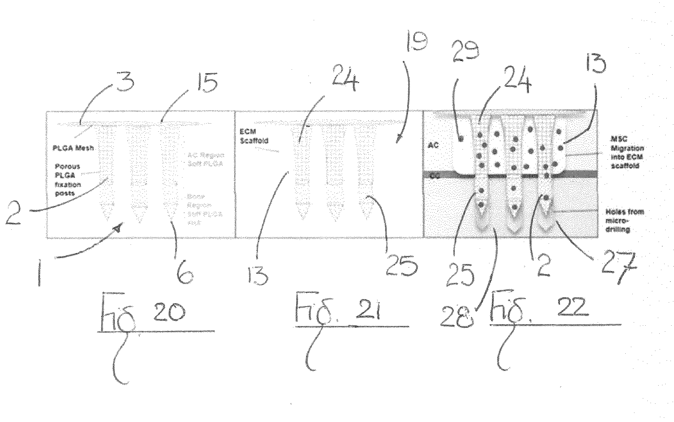

Figures 20 to 22 show another embodiment of the anchoring pin 1 of the invention in which the anchoring pin 1 is provided with three porous shafts 2 and the implant retainer 10 of the head 3 is mesh-like in structure. As shown in Figure 20, the porous shafts 2 are formed from tissue specific or compatible zones in the form of a cartilage region 24 towards the shaft proximal end 4 and a bone region 25 contiguous with the cartilage region 24 towards the distal end tip 6. The cartilage region 24 and the bone region 25 can be formed from biocompatible and biodegradable polymer materials having different physical, chemical or biological properties in accordance with tissue types such as different stiffnesses adapted for penetrating or insertion in the cartilage and bone tissues in which the shafts 2 are to be inserted. The tissue specific or compatible zones 24, 25 can therefore support spatially defined tissue development if desired.

For example, the anchoring pin 1 can be formed from porous poly(lactic-co-glycolic acid) (PLGA) which assists in augmenting the degradation rate of the anchoring pin 1 *in vivo.* In addition, cartilage region 24 can be formed from softer PLGA while the bone region 25 of the shafts 2 can be formed from stiffer or harder PLGA. Moreover, if desired, the polymers such as PLGA can also be coated, augmented or doped with other materials to alter the physical, chemical or biochemical properties of the anchoring pin. For example, the stiffer PLGA polymer of the bone region 25 can be doped with osteoinductive materials such as hydroxyapatite which also assists in stiffening the shafts 2.

In use and as shown particularly in Figures 5 to 12, the anchoring pin 1 is capable of securing an implant such as a porous scaffold 13 or engineered tissue 18 in place within a cartilage defect 14 - a process which would frequently otherwise involve complex suturing to fixate the implant. Where the implant is a scaffold 13, the anchoring pin 1 functions by holding the scaffold 13 into the defect 14 by means of the platform-like scaffold retainer 10 of the head 3 on the shaft 2. There is no need to screw the anchoring pin 1 into place as the anchoring pin 1 can be press-fit into a hole that is drilled into the subchondral bone and the like i.e. the anchoring pin 1 is implanted manually in a press-fit technique analogous to a 'thumb-tac' being pressed into a board.

The laterally extending ridges 7 present on the shaft 2 of the anchoring pin 1 allow tissue integration and ensure a more robust fixation of the anchoring pin 1 and any associated scaffold 13 within the bone.

Ensuring stem/progenitor cells remain in a site of injury is a prerequisite for a healing response and the effective application of a porous scaffold 13 as a means of sequestering these cells is an acknowledged method in regenerative medicine. Primarily, the scaffold 13 serves as a location where cells can adhere, proliferate, and excrete proteins which constitute the repair tissue. Ancillary to this the scaffold 13 can also provide cues which have been shown to improve the quality of the tissue formed by committing the stem cells to the necessary lineage (e.g. chondrocytes for cartilage regeneration). To this end, the use of the anchoring pin 1 to effectively fixate the ECM scaffold 13 in place in a defect 14 not only ensures that an appropriate environment for sequestering cells and supporting their growth is provided but also ensures optimal cell differentiation towards a chondrogenic phenotype for positive clinical outcomes.

The anchoring pin 1 is shaped and dimensioned to work in conjunction with microfracture or microdrilling which makes use of small holes or perforations 27 formed in subchondral bone 28 by picks or microdrilling to treat focal cartilage defects. However, by nature these holes 27 have a small diameter making traditional orthopaedic screws or pins inapplicable with the result that existing microfracture techniques are incompatible with known ECM scaffolds and the like while the medium-long term efficacy of this surgical approach is limited. However, the additive manufacturing production method for the anchoring pin 1 of the invention and the design of the anchoring pin 1 facilitates the generation of anchoring pins 1 that are within the range of microfracture diameters. Additionally, the diameter of the anchoring pins 1 is tuneable and hence the anchoring pin 1 can be conformed to any drill size used in microfracture techniques. In general, the anchoring pin 1 employed in a surgical technique such as microfracture/microdrilling will have a shaft 2 having a diameter less than that of the drill bit or pick e.g. about 1 to 2% smaller. Accordingly, for a 1.6mm drill bit typically employed in microdrilling a shaft diameter of about 1.58mm is suitable. More particularly, as indicated above, the relative dimensions of the shaft 2, head 3 and chamfer 8 can be varied as required in accordance with the dimensions of the holes 27 employed in microfracture and microdrilling techniques to securely hold ECM scaffolds 13 in place while at the same time preventing over insertion of the anchoring pin 1 in the holes 27 and ensuring optimal spacing of the head 3 from the bone 30 in which it is inserted so that the head 3 can be recessed or disposed parallel with respect to surrounding cartilage 31 to eliminate rubbing or friction between the head 3 and oppositely disposed adjacent tissue.

If desired, a surgeon can employ a template to position and pick or drill holes 27 at defect site so that implants such as ECM scaffolds can be precisely located at the defect as required prior to insertion of the anchoring pins 1 through the ECM scaffolds 13 to hold the ECM scaffolds 13 in place at the defects.

Moreover, by coupling the microfracture/microdrilling approach with a suitable scaffold 13 and the anchoring pin 1 of the invention a new method of regenerating cartilage in a patient results in which the overall clinical outcome is greatly enhanced. More particularly, where the anchoring pin 1 shown in Figures 9 to 12 is combined with the microfracture/microdrilling method, the function of the regular array structure 15 is twofold - the first being to provide individual microchamber cells 17 within which blood from microfracture holes can clot, trapping any stem cells from the patient (Figures 9 and 10). Secondly, the microchamber cells 17 can also function as a tissue-engineering scaffold for forming articular-like cartilage or other tissues *in vitro* (Figures 11 and 12) prior to implantation of the anchoring pin 1. Following tissue maturation in culture over a period of weeks, the engineered cartilage, which has integrated with the microchamber cells 17 in the head 3 of the anchoring pin 1, can then be implanted into a defect and held in place using the aforementioned press-fit technique of the anchoring pin 1.

More particularly, as shown in Figure 22, the anchoring pin 1 provides a fixation method with appropriate robustness and longevity to facilitate the repair of articular surfaces with an appropriate regenerative scaffold 13 without the need for alterations to the existing microfracture technique.

As indicated above, the anchoring pin 1 can be formed from any suitable biocompatible or biodegradable materials such as polymers or blends of polymers or the like e.g. polycaprolactone or PLGA. However, different polymers or blends of polymers can also be used to augment the degradation rate of the material. In addition, as described in the embodiment of Figures 20 to 22, the polymer material of the anchoring pin 1 can be combined with other materials including osteoinductive materials such as nano particles of hydroxyapatite to promote osteo-integration in the subchondral region of implants.

The anchoring pin 1, and in particular the shaft 2, is preferably porous as the porosity of the anchoring pin 1 serves to enhance cartilage regeneration and bone integration. The anchoring pin 1 can be fully or partially porous while lateral porosity is considered to be particularly beneficial for optimal results. The degree of porosity and pore size can be varied as required. The porosity of the anchoring pin 1 can be generated as a result of the materials from which the anchoring pin 1 is formed e.g. a porogen such as a salt can be incorporated into biocompatible polymer material used to form the anchoring pin 1 with the porogen being removed by washing following formation of the anchoring pin 1. Alternatively, the pores can be formed during fabrication using continuous additive manufacturing techniques such as 3D printing as discussed further below.

In another embodiment, the anchoring pins 1 may also be fabricated to contain solubilised bone and/or articular cartilage ECM within the (PCL/PLGA) polymer materials. The addition of the ECM within the anchoring pins 1 aids cell attachment to the anchoring pins 1 and promotes integration with native subchondral bone.

In another embodiment, as also shown in Figure 22, the homing of MSCs 29 from the bone marrow beneath a scaffold 13 can be enhanced by the incorporation of transforming growth factor (TGF)-β3, a potent chemoattractant, into the anchoring pin 1 or the scaffold 13 used in combination with the anchoring pin 1.

The anchoring pin 1 of the invention can be fabricated using continuous additive manufacturing techniques such as 3D printing. Accordingly, the anchoring pin 1 can be sized and spatially modified with ease in accordance with the desired application e.g. the apertured head 3 can be formed with the relatively large apertures 26 or the mesh/regular array structure 15 or can be sized for use with microfracture treatment methods. As a result, the shaft 2 and head 3 of the anchoring pin 1 are formed within a single structure within a single production step. Moreover, additional features, geometries, and components can be added to the anchoring pin 1 during manufacture without the need for post fabrication modification of the structure.

## Claims

1. A biocompatible implant anchoring pin comprising:
a shaft for insertion in tissue and
an integral head on the shaft configured to retain an implant in tissue.

2. A biocompatible implant anchoring pin as claimed in Claim 1 wherein the head comprises an implant retainer.

3. A biocompatible implant anchoring pin as claimed in Claim 2 wherein the implant retainer comprises a scaffold implant retainer.

4. A biocompatible implant anchoring pin as claimed in Claim 2 or Claim 3 wherein the implant retainer comprises an engineered tissue implant retainer.

5. A biocompatible implant anchoring pin as claimed in any of Claims 2 to 4 wherein the implant retainer is apertured.

6. A biocompatible implant anchoring pin as claimed in Claim 5 wherein the apertures are defined by a regular array.

7. A biocompatible implant anchoring pin as claimed in any of Claims 1 to 6 wherein the shaft comprises tissue grips.

8. A biocompatible implant anchoring pin as claimed Claim 7 wherein the tissue grips comprise laterally extending ridges.

9. A biocompatible implant anchoring pin as claimed in any of Claims 1 to 8 wherein the anchoring pin comprises a spacer to space the integral head from tissue.

10. A biocompatible implant anchoring pin as claimed in Claim 9 wherein the spacer comprises a chamfer on the shaft.

11. A biocompatible implant anchoring pin as claimed in any of Claims 1 to 10 wherein the shaft comprises tissue specific or tailored zones having different physical, chemical or biological properties in accordance with tissue type.

12. A biocompatible implant anchoring pin as claimed in any of Claims 1 to 11 wherein the anchoring pin is porous.

13. An implant retention system comprising an anchoring pin as claimed in any of Claims 1 to 12 combined with an implant.

14. An implant retention system as claimed in Claim 13 wherein the implant comprises a scaffold.

15. An implant retention system as claimed in Claim 14 wherein the anchoring pin is combined with the scaffold by insertion of the anchoring pin in the scaffold or is combined with the scaffold in an integrated unitary structure.
